# EUROPÄISCHE PATENTANMELDUNG

(11) **EP 3 349 219 A1**
(43) Veröffentlichungstag der Anmeldung: **18.07.2018**
(21) Anmeldenummer: 17194001.8
(22) Anmeldetag: 29.09.2017
(51) Int. Cl.: G16H 40/63, G16H 50/70

(54) **VERFAHREN UND SYSTEM ZUM ERMITTELN EINES UNTERSUCHUNGSPARAMETERS FÜR EINE MEDIZINISCHE BILDGEBUNGSUNTERSUCHUNG**

(71) Anmelder: Siemens Healthcare GmbH, 91052 Erlangen (DE)
(72) Erfinder: Allmendinger, Thomas, 91301 Forchheim (DE); Baer, Matthias, 91054 Erlangen (DE); Feuerlein, Ute, 91052 Erlangen (DE); Haberland, Ulrike, 91052 Erlangen (DE); Huber, Peter, 91575 Windsbach (DE); Hölzer, Philipp, 91088 Bubenreuth (DE); Koch, Christiane, 91330 Eggolsheim (DE); Raupach, Rainer, 91336 Heroldsbach (DE); Schmidt, Sebastian, 91085 Weisendorf (DE)

(57) **Zusammenfassung**

Die Erfindung betrifft ein Verfahren zum Ermitteln eines Untersuchungsparameters für eine medizinische Bildgebungsuntersuchung eines Patienten, insbesondere mittels einer medizinischen Bildgebungsvorrichtung, wobei das Verfahren die folgenden Schritte umfasst:
- Bereitstellen von Untersuchungsauftragsdaten, welche die medizinische Bildgebungsuntersuchung des Patienten betreffen,
- Generieren einer Datenbankanfrage für eine klinische Datenbank, wobei die klinische Datenbank klinische Daten des Patienten umfasst, basierend auf den Untersuchungsauftragsdaten,
- Bereitstellen der klinischen Daten des Patienten basierend auf der Datenbankanfrage,
- Ermitteln des Untersuchungsparameters basierend auf den klinischen Daten.

## Beschreibung

Die Erfindung betrifft ein Verfahren und ein System zum Ermitteln eines Untersuchungsparameters für eine medizinische Bildgebungsuntersuchung eines Patienten. Die Erfindung betrifft ferner ein Computerprogrammprodukt und ein computerlesbares Medium.

Bei der Durchführung einer medizinischen Bildgebungsuntersuchung, zum Beispiel mittels Computertomographie, Magnetresonanztomographie oder Röntgen, muss eine Vielzahl von technischen Parametern eingestellt werden, um ein für die jeweilige Fragestellung optimales Ergebnis zu erhalten. Beispiele dafür sind Parameter, welche eine Röhrenspannung, einen Röhrenstrom, Kernspin-Echozeiten, Rekonstruktionsverfahren, Bildnachverarbeitungsverfahren oder ähnliches betreffen. Dazu gibt es Benutzeroberflächen, welche die Eingabe dieser Parameter ermöglichen. Heutzutage werden dazu meistens Computer oder Touchscreens verwendet, früher waren Stellräder und ähnliches gebräuchlich.

Dabei stellt sich das Problem, dass es weniger erfahrenen Benutzern oft nicht ohne weiteres möglich ist, die optimalen Parameter für die jeweilige Untersuchung festzulegen, weil das eine intensive Beschäftigung mit der Materie und eine entsprechende Ausbildung erfordert. Als Folge finden immer wieder Untersuchungen mit nicht-optimalen Parametern statt, die dann entweder eine höhere Strahlendosis oder eine nichtoptimale Bildqualität aufweisen. Dies ist speziell ein Problem, wenn das Gerät durch Personen bedient werden muss, die in der Bedienung dieses speziellen Geräts wenig erfahren sind, zum Beispiel im Notdienst. Gleichzeitig stehen immer mehr bildgebende Geräte in einem Umfeld, in dem es schwierig ist, immer Fachpersonal vor Ort zu haben, zum Beispiel in abgelegenen Zentren, der Notaufnahme oder im mobilen Einsatz.

US 7355406 B2 offenbart ein Verfahren zur Planung einer Untersuchung eines Untersuchungsobjekts in einer Magnetresonanzanlage, wobei Bilder von unterschiedlichen Bereichen des Untersuchungsobjekts aufgenommen werden, die zu einem Gesamtbild zusammengesetzt werden.

US 8000510 B2 offenbart ein Verfahren zur Steuerung eines Schnittbildaufnahmesystems, bei dem ein Scanprotokoll aus einer Anzahl von Scanprotokollen ausgewählt wird.

US 8401872 B2 offenbart ein Verfahren zum Betreiben einer medizinischen Diagnoseeinrichtung, mit deren Hilfe medizinische Fragestellungen beantwortet werden sollen.

US 8687762 B2 offenbart ein CT-System mit einem Computersystem, wobei im Computersystem eine Auswerteeinheit für einen vorgegebenen logischen Entscheidungsbaum integriert ist.

US 9636077 B2 offenbart ein Verfahren zur automatischen Auswahl eines Scanprotokolls zur tomographischen Aufnahme eines Röntgenbildes eines Patienten.

Die Erfindung hat die Aufgabe, eine verbesserte Ermittlung von Untersuchungsparametern für eine medizinische Bildgebungsuntersuchung zu ermöglichen. Jeder der Gegenstände der unabhängigen Ansprüche löst jeweils diese Aufgabe. In den abhängigen Ansprüchen sind weitere vorteilhafte Aspekte der Erfindung berücksichtigt.

Die Erfindung betrifft ein Verfahren zum Ermitteln eines Untersuchungsparameters für eine medizinische Bildgebungsuntersuchung eines Patienten, insbesondere mittels einer medizinischen Bildgebungsvorrichtung, wobei das Verfahren die folgenden Schritte umfasst:
- Bereitstellen von Untersuchungsauftragsdaten, welche die medizinische Bildgebungsuntersuchung des Patienten betreffen,
- Generieren, insbesondere automatisches Generieren, einer Datenbankanfrage für eine klinische Datenbank basierend auf den Untersuchungsauftragsdaten, wobei die klinische Datenbank klinische Daten des Patienten umfasst,

- Bereitstellen der klinischen Daten des Patienten basierend auf der Datenbankanfrage,
- Ermitteln des Untersuchungsparameters basierend auf den klinischen Daten.

Die Untersuchungsauftragsdaten können beispielsweise in einem Radiologie-Informationssystem hinterlegt sein, insbesondere in Form eines Eintrages in einer radiologischen Worklist. Die Untersuchungsauftragsdaten können insbesondere Patientenidentifikationsdaten, beispielsweise einen Patientennamen, eine Identifikationsnummer und/oder ein Geburtsdatum umfassen. Die Untersuchungsauftragsdaten können insbesondere Informationen zur Bildgebungsmodalität und/oder zu einem zu untersuchenden Körperbereich, insbesondere zu einem zu untersuchenden Organ umfassen. Die Untersuchungsauftragsdaten können insbesondere Informationen und/oder eine Fragestellung, in Form eines unstrukturierten Texts, beispielsweise als Freitext, umfassen. Beispielsweise können die Untersuchungsauftragsdaten die Zeichenkette "CT Kopf" aufweisen.

Die klinischen Daten des Patienten können beispielsweise zumindest teilweise in einer elektronischen Patientenakte des Patienten hinterlegt sein. Die klinische Datenbank kann insbesondere von einem Krankenhausinformationssystem gebildet sein und/oder in einer zentral oder verteilt gespeicherten Form, insbesondere in einer Cloud, oder in einer beliebigen anderen elektronischen Form vorliegen. Die Datenbankanfrage kann insbesondere an die klinische Datenbank gesendet werden, wobei beispielsweise Verfahren zur Authentifizierung und/oder Verschlüsselung der Datenbankanfrage verwendet werden können. Die klinischen Daten des Patienten können insbesondere basierend auf der Datenbankanfrage aus einer Vielzahl von klinischen Daten verschiedener Patienten und/oder als Teilmenge einer größeren klinischen Datenmenge des Patienten in der klinischen Datenbank ausgewählt werden und/oder in Form einer Query bereitgestellt werden. Die klinischen Daten des Patienten können insbesondere von der klinischen Datenbank übertragen werden, wobei beispielsweise Verfahren zur Authentifizierung und/oder Verschlüsselung der klinischen Daten verwendet werden können.

Die klinischen Daten des Patienten können insbesondere zumindest teilweise in Form von Abrechnungsdaten, zum Beispiel von Krankenkassen, hinterlegt sein. Die Abrechnungsdaten können zum Beispiel frühere medizinische Bildgebungsuntersuchungen des Patienten und/oder Medikamente, die dem Patienten verschrieben und/oder von dem Patienten eingenommen wurden, betreffen.

Die klinischen Daten des Patienten können beispielsweise zumindest einen Patientenparameter aufweisen, welcher beispielsweise einen Aspekt oder mehrere Aspekte eines Zustands des mit der medizinischen Bildgebungsuntersuchung zu untersuchenden Patienten betrifft. Bei den Aspekten des Zustands des Patienten kann es sich insbesondere um demografische, physiologische und/oder ethnische Aspekte handeln.

Ein Patientenparameter kann beispielsweise eine Herzrate, eine Herzratenvariabilität, eine Dicke oder eine Schwächung der Röntgenstrahlung in bestimmten Bereichen des Körpers des Patienten und/oder in bestimmten Projektionsrichtungen, ein Alter, ein Geschlecht, ein Gewicht, eine Größe, einen Body-Mass-Index, Laborwerte, beispielsweise einen Kreatinin-Wert, eine Dichte oder eine Konzentration eines Stoffes im Körper des Patienten oder eine daraus abgeleitete Größe, eine Kooperationsbereitschaft, eine Historie, eine Diagnose, eine Anamnese oder ähnliches oder Kombinationen davon betreffen. Beispielsweise kann mit einem Patientenparameter, der die Kooperationsbereitschaft des Patienten betrifft, angegeben werden, dass der Patienten nicht kooperativ ist. Beispielsweise kann mit einem Patientenparameter, der die Anamnese des Patienten betrifft, angegeben, dass der Patienten vor kurzem einen Schlaganfall hatte. Beispielsweise kann ein Patientenparameter, der eine Diagnose des Patienten betrifft, einen Klassifikations-Code, insbesondere in Form eines ICD-Codes der International Statistical Classification of Diseases and Related Health Problems, umfassen.

Eine Ausführungsform der Erfindung sieht vor, dass der Untersuchungsparameter aus der Gruppe gewählt ist, welche aus einem Akquisitionsparameter, einem Rekonstruktionsparameter, einem Bildverarbeitungsparameter, einem Kontrastmittelparameter und Kombinationen davon besteht.

Bei dem Untersuchungsparameter kann es sich insbesondere um einen Untersuchungsprotokollparameter handeln. Ein Untersuchungsprotokollparameter kann insbesondere ein Akquisitionsparameter sein. Ein Akquisitionsparameter kann beispielsweise eine Röhrenspannung, einen Röhrenstrom, eine Rotationszeit, einen Spiralpitch, einen oder mehrere Trigger-Zeitpunkte für eine Röhrenstrommodulation im Herzzyklus oder ähnliches oder Kombinationen davon betreffen. Ein Untersuchungsprotokollparameter kann insbesondere ein Rekonstruktionsparameter sein. Ein Rekonstruktionsparameter kann beispielsweise ein Faltungskern, einen Faltungsalgorithmus, eine Schichtdicke, ein Schichtinkrement oder ähnliches oder Kombinationen davon betreffen. Ein Untersuchungsprotokollparameter kann insbesondere ein Kontrastmittelparameter sein. Ein Kontrastmittelparameter kann beispielsweise eine Kontrastmittelmenge, eine Flussrate oder ähnliches oder Kombinationen davon betreffen. Im Unterschied zu den klinischen Daten kann es sich bei dem Untersuchungsparameter insbesondere um einen im Wesentlichen technischen Parameter handeln. Der Untersuchungsparameter kann beispielsweise mittels einer Ausgabeeinheit ausgegeben werden, insbesondere auf einem Bildschirm angezeigt werden und/oder in einem Speicher zur weiteren Verarbeitung gespeichert werden und/oder an eine Komponente der medizinischen Bildgebungsvorrichtung übertragen werden.

Die klinischen Daten des Patienten können insbesondere eine Information zu Nieren des Patienten, insbesondere in Bezug auf Kreatinin, eine glomeruläre Filtrationsrate (GFR) und/oder eine Inulin-Clearance, umfassen. Basierend auf der Information zu den Nieren des Patienten kann beispielsweise ein Kontrastmittelparameter, welcher eine Menge des Kontrastmittels betrifft, und/oder ein Untersuchungsparameter, welcher einen zeitlichen Ablauf (Timing) einer BilddatenAkquisition mittels einer medizinischen Bildgebungsvorrichtung betrifft, ermittelt werden. Dabei kann die medizinische Bildgebungsuntersuchung insbesondere die Nieren und/oder die Harnwege des Patienten betreffen.

Die klinischen Daten des Patienten können insbesondere eine Information zu einer Allergie des Patienten umfassen. Basierend auf der Information zu der Allergie des Patienten kann beispielsweise ein Kontrastmittelparameter ermittelt werden.

Die klinischen Daten des Patienten können insbesondere eine Information zu einer Herzleistung des Patienten, insbesondere in Bezug auf eine Ejektionsfraktion, einen Blutdruck, eine Klappenfunktion und/oder Ultraschallaufnahmen des Herzens des Patienten, umfassen. Basierend auf der Information zu der Herzleistung des Patienten kann beispielsweise ein Untersuchungsparameter, der das Kontrastmittel-Bolus-Timing betrifft, ermittelt werden.

Die klinischen Daten des Patienten können insbesondere eine Information zu einem Gewicht des Patienten umfassen. Basierend auf der Information zu dem Gewicht des Patienten kann beispielsweise ein Kontrastmittelparameter, welcher eine Menge des Kontrastmittels betrifft, ermittelt werden.

Die klinischen Daten des Patienten können insbesondere eine Information zu einem Herzrhythmus des Patienten, insbesondere in Bezug auf ein Elektrokardiogramm (EKG), einen Befund und/oder eine klinische Untersuchung des Herzrhythmus, umfassen. Basierend auf der Information zu dem Herzrhythmus des Patienten kann beispielsweise ein Untersuchungsparameter, welcher ein Kontrastmittel-Timing betrifft, und/oder ein Untersuchungsparameter, welcher ein Gating einer BilddatenAkquisition mittels einer medizinischen Bildgebungsvorrichtung betrifft, ermittelt werden. Dabei kann die medizinische Bildgebungsuntersuchung insbesondere das Herz des Patienten betreffen.

Die klinischen Daten des Patienten können insbesondere eine Information zu einer Lungenfunktion des Patienten, insbesondere in Bezug auf Spirometrie und/oder eine klinische Untersuchung der Lungenfunktion, umfassen. Basierend auf der Information zu der Lungenfunktion des Patienten kann beispielsweise ein Untersuchungsparameter, welcher eine geplante Atemanhaltezeit des Patienten während der medizinischen Bildgebungsuntersuchung betrifft, ermittelt werden.

Die klinischen Daten des Patienten können insbesondere eine Information zu einer Diagnose des Patienten, insbesondere in Bezug auf eine akut lebensbedrohliche Diagnose, umfassen. Basierend auf der Information zu der Diagnose des Patienten kann beispielsweise ein Untersuchungsparameter, welcher Planung und/oder eine Dauer der medizinischen Bildgebungsuntersuchung betrifft, ermittelt werden, insbesondere derart ermittelt werden, dass die Dauer der medizinischen Bildgebungsuntersuchung möglichst kurz ist.

Die klinischen Daten des Patienten können insbesondere eine Information zu einem Benutzer, insbesondere in Bezug auf medizinisches Personal, umfassen. Die klinischen Daten des Patienten können beispielsweise Informationen zu einem Benutzer, insbesondere einem Arzt, welcher den Patienten behandelt hat und/oder welcher den Patienten der medizinischen Bildgebungsuntersuchung zugewiesen hat, umfassen. Basierend auf der Information zu dem Benutzer kann beispielsweise ein Untersuchungsparameter ermittelt werden, mit dem die medizinische Bildgebungsuntersuchung an eine Präferenz des Benutzers angepasst werden kann. Beispielsweise kann der auf diese Weise ermittelte Untersuchungsparameter entsprechend der Präferenz des Benutzers eine Angabe darüber umfassen, ob bei der medizinischen Bildgebungsuntersuchung eine Neuroperfusion durchzuführen ist oder nicht.

Die klinischen Daten des Patienten können insbesondere eine Information zu einer Schwangerschaft des Patienten umfassen. Basierend auf der Information zu der Schwangerschaft des Patienten kann beispielsweise ein Untersuchungsparameter ermittelt werden, der eine Angabe darüber umfasst, ob bei der medizinischen Bildgebungsuntersuchung eine ionisierende Strahlung verwendet werden kann oder nicht.

Die klinischen Daten des Patienten können insbesondere eine Information zu einer Erkrankung einer Sinneswahrnehmung des Patienten, insbesondere in Bezug auf eine Taubheit und/oder eine sensorische Aphasie, umfassen. Basierend auf der Information zu der Erkrankung der Sinneswahrnehmung des Patienten kann beispielsweise ein Untersuchungsparameter, welcher eine Instruktion des Patienten, insbesondere in Bezug auf dessen Atmung, betrifft. Beispielsweise kann bei Taubheit des Patienten der Untersuchungsparameter eine Angabe darüber umfassen, dass keine Atemkommandos verwendet werden soll und/oder dass eine Bilddatenakquisition während einer freien Atmung des Patienten erfolgen soll (free breathing mode).

Die klinischen Daten des Patienten können insbesondere eine Information zu einer onkologischen Vorerkrankung des Patienten umfassen. Basierend auf der Information zu der onkologischen Vorerkrankung des Patienten kann beispielsweise ein Untersuchungsparameter, welcher eine Auswahl zumindest eines Bildgebungsverfahrens für die medizinische Bildgebungsuntersuchung betrifft, ermittelt werden. Beispielsweise können bei einer onkologischen Vorerkrankung des Patienten zusätzliche Bildgebungsverfahren ausgewählt werden, so dass die medizinische Bildgebungsuntersuchung zum Beispiel sowohl eine Bildgebungsuntersuchung mittels Computertomographie als auch eine Bildgebungsuntersuchung mittels Magnetresonanztomographie (MRT), insbesondere Diffusions-MRT, umfasst.

Die klinischen Daten des Patienten können insbesondere eine Information zu einer geplanten Intervention an dem Patienten, insbesondere in Bezug auf eine Operation, eine radiologische Interventionen und/oder eine Strahlentherapie, umfassen. Basierend auf der Information zu der geplanten Intervention an dem Patienten kann beispielsweise ein Untersuchungsparameter eines Untersuchungsprotokolls für eine Planung der Intervention basierend auf der medizinischen Bildgebungsuntersuchung ermittelt werden.

Die klinischen Daten des Patienten können insbesondere eine Information zu Zeitpunkten, zu denen bestimmte Informationen zum Patienten, zum Beispiel verschiedene Diagnosen, generiert und/oder in die klinische Datenbank aufgenommen wurden, umfassen. Basierend auf der Information zu den Zeitpunkten kann ein Untersuchungsparameter, welcher eine Dringlichkeit der medizinischen Bildgebungsuntersuchung und/oder Dringlichkeiten von Teiluntersuchungen der medizinischen Bildgebungsuntersuchung betrifft, ermittelt werden. Beispielsweise kann einer Teiluntersuchung, welche eine zeitlich relativ kurz zurückliegende Diagnose betrifft, eine höhere Dringlichkeit zugeordnet werden als einer Teiluntersuchung, welche eine zeitlich relativ lang zurückliegende Diagnose betrifft.

Eine Information in den klinischen Daten kann beispielweise eine strukturierte Information, insbesondere einen Wert und/oder eine Zuordnung zu einer Klasse aus einer Mehrzahl von Klassen, und/oder eine unstrukturierte Information, beispielsweise in Form eines Freitexts, aufweisen.
Eine Ausführungsform der Erfindung sieht vor, dass die Datenbankanfrage für eine unscharfe Suche in der klinischen Datenbank geeignet ist. Insbesondere kann basierend auf der Datenbankanfrage eine Suche, beispielsweise eine unscharfe Suche, in der klinischen Datenbank ausgeführt werden, wobei beispielsweise die klinischen Daten des Patienten gefunden werden können.

Auf diese Weise können die klinischen Daten des Patienten selbst dann in der klinischen Datenbank ausgewählt und bereitgestellt werden, wenn beispielsweise der Patientenname im Untersuchungsauftrag von dem entsprechenden Patientennamen in der klinischen Datenbank abweicht. Die Datenbankanfrage kann beispielsweise mittels einer Datenbanksprache, insbesondere mittels einer Datenbank-Anfragesprache, formuliert sein und/oder zur Filterung von Daten aus der klinischen Datenbank geeignet sein. Die Datenbankanfrage kann insbesondere zumindest einen regulären Ausdruck umfassen. Die Datenbankanfrage für die klinische Datenbank kann insbesondere automatisch, beispielsweise ohne Benutzer-Interaktion oder ohne wesentliche Benutzer-Interaktion, basierend auf den Untersuchungsauftragsdaten generiert werden. Die Datenbankanfrage für die klinische Datenbank kann insbesondere basierend auf den Untersuchungsauftragsdaten in einem Prozessor generiert werden, beispielsweise indem der Prozessor Berechnungen basierend auf den Untersuchungsauftragsdaten ausführt. Insbesondere können die Untersuchungsauftragsdaten in den Prozessor geladen werden.

Die klinische Datenbank kann insbesondere zusätzlich zu den klinischen Daten des Patienten weitere klinische Daten des Patienten aufweisen. Insbesondere kann eine weitere Datenbankanfrage für die klinische Datenbank basierend auf den Untersuchungsauftragsdaten und/oder basierend auf der Datenbankanfrage und/oder basierend auf den klinischen Daten generiert werden, insbesondere automatisch generiert werden. Basierend auf der weiteren Datenbankanfrage können weitere klinische Daten des Patienten bereitgestellt werden. Die weiteren klinischen Daten des Patienten können beispielsweise eine Obermenge oder eine Teilmenge der klinischen Daten des Patienten bilden. Die weiteren klinischen Daten können beispielsweise eine Teilmenge der klinischen Daten und/oder in den klinischen Daten nicht vorhandene Daten umfassen. Der Untersuchungsparameter kann insbesondere basierend auf den weiteren klinischen Daten ermittelt werden, insbesondere nach einem oder mehreren der Aspekte, die in dieser Anmeldung im Zusammenhang mit den klinischen Daten offenbart sind, ermittelt werden.

Insbesondere können Sensordaten des Patienten bereitgestellt werden. Die Sensordaten des Patienten können beispielsweise mittels einer Sensoreinheit erfasst werden. Die Sensoreinheit kann beispielsweise ein optisches Signal, welches den Patienten betrifft, und/oder ein bioelektrisches Signal, welches den Patienten betrifft, erfassen. Das optische Signal kann beispielsweise auf Licht, welches von dem Patienten reflektiert wird, basieren. Das bioelektrische Signal kann beispielsweise auf elektrischen Potentialen an elektrischen Kontakten, die mit dem Körper des Patienten elektrisch leitend verbunden sind, basieren. Die Sensoreinheit kann insbesondere ein Elektrokardiogramm-Gerät und/oder eine Kamera, beispielsweise eine 3D-Kamera, sein und/oder aufweisen. Die Sensordaten können insbesondere Elektrokardiogrammdaten und/oder Kamerabilddaten, insbesondere 3D-Kamerabilddaten, umfassen.

Die Sensoreinheit kann ferner zum Ermitteln eines Signalverarbeitungsergebnisses basierend auf einem Signal, welches mittels der Sensoreinheit erfasst wird, ausgebildet sein. Die Sensordaten können insbesondere zumindest ein Signalverarbeitungsergebnis umfassen, welches basierend auf einem Signal, welches mittels der Sensoreinheit erfasst wurde, ermittelt wird. Bei Kamerabilddaten kann ein Signalverarbeitungsergebnis beispielsweise eine Abmessung des Patienten und/oder eine Position und/oder Orientierung des Patienten, insbesondere relativ zu der medizinischen Bildgebungsvorrichtung, und/oder eine Biometrie des Patienten, insbesondere eine Gesichtserkennung des Patienten, betreffen.

Beispielsweise kann die Datenbankanfrage für die klinische Datenbank basierend auf den Untersuchungsauftragsdaten und basierend auf den Sensordaten generiert werden, insbesondere automatisch und/oder basierend auf einem trainierten Algorithmus zum Generieren der Datenbankanfrage generiert werden. Beispielsweise können die klinischen Daten basierend auf den Sensordaten klassifiziert und/oder strukturiert werden, insbesondere automatisch und/oder basierend auf einem trainierten Algorithmus zum Klassifizieren und/oder Strukturieren der klinischen Daten klassifiziert und/oder strukturiert werden. Beispielsweise kann der Untersuchungsparameter basierend auf den klinischen Daten und basierend auf den Sensordaten ermittelt werden, insbesondere automatisch und/oder basierend auf einem trainierten Untersuchungsparameter-Ermittlungs-Algorithmus ermittelt werden. Auf diese Weise kann das Generieren der Datenbankanfrage und/oder das Klassifizieren und/oder Strukturieren der klinischen Daten und/oder das Ermitteln des Untersuchungsparameters in Bezug auf einen Zustand des Patienten optimiert werden, insbesondere an den Zustand des Patienten in Echtzeit angepasst werden.

Eine Ausführungsform der Erfindung sieht vor, dass die Datenbankanfrage ferner basierend auf künstlicher Intelligenz generiert wird. Die Datenbankanfrage kann insbesondere basierend auf einem trainierten Algorithmus zum Generieren der Datenbankanfrage generiert werden. Der trainierte Algorithmus zum Generieren der Datenbankanfrage kann insbesondere die Untersuchungsauftragsdaten und/oder die Sensordaten verarbeiten, wobei die Datenbankanfrage generiert wird.

Eine Ausführungsform der Erfindung sieht vor, dass die klinischen Daten basierend auf künstlicher Intelligenz strukturiert und/oder klassifiziert werden. Die klinischen Daten können insbesondere basierend auf einem trainierten Algorithmus zum Klassifizieren und/oder Strukturieren der klinischen Daten strukturiert und/oder klassifiziert werden. Der trainierte Algorithmus zum Klassifizieren und/oder Strukturieren der klinischen Daten kann insbesondere die klinischen Daten und/oder die Sensordaten verarbeiten, wobei die strukturierten und/oder klassifizierten klinische Daten des Patienten erzeugt werden können. Auf diese Weise kann ein Patientenparameter, der beispielsweise eine Diagnose betrifft, basierend auf einem Befund, der als unstrukturierter Text, insbesondere Fließtext, vorliegt, ermittelt werden.

Eine Ausführungsform der Erfindung sieht vor, dass der Untersuchungsparameter basierend auf den klinischen Daten mittels eines trainierten Untersuchungsparameter-Ermittlungs-Algorithmus, welcher auf maschinellem Lernen basiert, ermittelt wird. Der Untersuchungsparameter-Ermittlungs-Algorithmus kann zum Beispiel mit Trainingsdaten, welche von erfahrenen Anwendern erstellt wurden, basierend auf maschinellem Lernen trainiert werden. Die Trainingsdaten können beispielsweise eine Vielzahl von Trainingspaaren aufweisen, wobei jedes Trainingspaar einen Trainingsinput mit klinischen Daten und einen Trainingsoutput mit Untersuchungsparametern aufweist. Alternativ oder zusätzlich können beim Ermitteln des Untersuchungsparameters basierend auf den klinischen Daten regelbasierte Verfahren verwendet werden.

Alternativ oder zusätzlich zum Ermitteln des Untersuchungsparameters basierend auf den klinischen Daten kann basierend auf den klinischen Daten und/oder basierend auf den Untersuchungsauftragsdaten und/oder basierend auf dem Untersuchungsparameter überprüft werden, ob die medizinische Bildgebungsuntersuchung angemessen ist und/oder ob zumindest eine Kontraindikation vorliegt, die einer Durchführung der medizinischen Bildgebungsuntersuchung entgegensteht. Dabei kann ebenfalls künstliche Intelligenz, insbesondere basierend auf maschinellem Lernen, verwendet werden.

Insbesondere ist hiermit ein Verfahren zur Überprüfung in Bezug auf Kontraindikationen, die einer medizinischen Bildgebungsuntersuchung eines Patienten entgegenstehen, offenbart, wobei das Verfahren die folgenden Schritte umfasst:
- Bereitstellen von Untersuchungsauftragsdaten, welche die medizinische Bildgebungsuntersuchung des Patienten betreffen,
- Generieren einer Datenbankanfrage für eine klinische Datenbank, welche klinische Daten des Patienten umfasst, basierend auf den Untersuchungsauftragsdaten,
- Bereitstellen der klinischen Daten des Patienten basierend auf der Datenbankanfrage,
- Überprüfen, ob zumindest eine Kontraindikation vorliegt, die einer Durchführung der medizinischen Bildgebungsuntersuchung entgegensteht, basierend auf den klinischen Daten und/oder basierend auf den Untersuchungsauftragsdaten.

Eine Ausführungsform der Erfindung sieht vor,
- dass die medizinische Bildgebungsuntersuchung des Patienten basierend auf dem Untersuchungsparameter durchgeführt wird,
- dass ein Untersuchungsergebnis der medizinischen Bildgebungsuntersuchung des Patienten erzeugt wird,
- dass ein Trainingsdatensatz mit einem Trainingsinput und einem Trainingsoutput zum Trainieren eines Maschinenlernalgorithmus erzeugt wird,
- dass der Trainingsinput die klinischen Daten und/oder die strukturierten und/oder klassifizierten klinischen Daten aufweist,
- dass der Trainingsoutput das Untersuchungsergebnis aufweist.

Der Trainingsinput kann ferner die Untersuchungsauftragsdaten und/oder die Sensordaten aufweisen. Eine Ausführungsform der Erfindung sieht vor, dass basierend auf dem Trainingsdatensatz ein Maschinenlernalgorithmus trainiert wird, welcher zum Generieren der Datenbankanfrage für die klinische Datenbank basierend auf den Untersuchungsauftragsdaten und/oder den Sensordaten und/oder zum Strukturieren und/oder Klassifizieren der klinischen Daten, insbesondere basierend auf den Sensordaten, und/oder zum Ermitteln des Untersuchungsparameters basierend auf den klinischen Daten und/oder den Sensordaten ausgebildet ist. Insbesondere kann auf diese Weise der Untersuchungsparameter-Ermittlungs-Algorithmus und/oder ein Algorithmus nach einem oder mehreren der in dieser Anmeldung offenbarten Aspekte, der zum maschinellen Lernen ausgebildet ist, trainiert werden. Insbesondere kann eine Nutzenfunktion für ein bestärkendes maschinelles Lernverfahren basierend auf einer Eingabe des Benutzers ausgewählt werden. Insbesondere kann der Maschinenlernalgorithmus basierend auf der Nutzenfunktion und dem bestärkenden maschinellen Lernverfahren trainiert werden.

Eine Ausführungsform der Erfindung sieht vor,
- dass zumindest ein Abrechenbarkeitskriterium bereitgestellt wird,
- dass basierend auf den Untersuchungsauftragsdaten und/oder basierend auf den klinischen Daten und/oder basierend auf den strukturierten und/oder klassifizierten klinischen Daten und/oder basierend auf dem Untersuchungsparameter eine Überprüfung der medizinischen Bildgebungsuntersuchung in Bezug auf das zumindest eine Abrechenbarkeitskriterium durchgeführt wird.

Das zumindest eine Abrechenbarkeitskriterium kann beispielsweise in dem Untersuchungsauftrag und/oder in der klinischen Datenbank und/oder in einer separaten Datenbank enthalten sein. Das zumindest eine Abrechenbarkeitskriterium kann unabhängig von dem Patienten sein oder in Abhängigkeit von dem Patienten, insbesondere in Abhängigkeit von dem Patienten zugeordneten Krankenkassendaten, ausgewählt werden.

Basierend auf der Überprüfung der medizinischen Bildgebungsuntersuchung in Bezug auf das zumindest eine Abrechenbarkeitskriterium kann ein Abrechenbarkeitsüberprüfungsergebnis, beispielsweise in Form einer Bildschirmanzeige und/oder in Form eines Datenbankeintrages, ausgegeben werden. Das Abrechenbarkeitsüberprüfungsergebnis kann insbesondere eine Angabe darüber, ob die medizinische Bildgebungsuntersuchung des Patienten abrechenbar ist oder nicht, und/oder zumindest ein Kriterium, welches für eine Abrechnung zu erfüllen ist, aufweisen.

Bei der Überprüfung der medizinischen Bildgebungsuntersuchung in Bezug auf das zumindest eine Abrechenbarkeitskriterium kann ebenfalls künstliche Intelligenz, insbesondere in Form von maschinellem Lernen mit Hilfe von Abrechenbarkeits-Trainingsdaten, verwendet werden. Ein Trainingsinput eines Trainingspaares der Abrechenbarkeits-Trainingsdaten kann insbesondere die Untersuchungsauftragsdaten und/oder die klinischen Daten und/oder die Sensordaten und/oder die strukturierten und/oder klassifizierten klinischen Daten und/oder den Untersuchungsparameter und/oder das zumindest eine Abrechenbarkeitskriterium aufweisen. Ein Trainingsoutput eines Trainingspaares der Abrechenbarkeits-Trainingsdaten kann insbesondere eine Abrechnungsinformation, beispielsweise darüber, ob die medizinische Bildgebungsuntersuchung des Patienten abrechenbar ist oder nicht, aufweisen.

Die Erfindung betrifft ferner ein System zum Ermitteln eines Untersuchungsparameters für eine medizinische Bildgebungsuntersuchung eines Patienten, insbesondere mittels einer medizinischen Bildgebungsvorrichtung, aufweisend:
- eine Untersuchungsauftrag-Bereitstellungseinheit, ausgebildet zum Bereitstellen von Untersuchungsauftragsdaten, welche die medizinische Bildgebungsuntersuchung des Patienten betreffen,
- eine Generierungseinheit, ausgebildet zum Generieren, insbesondere zum automatischen Generieren, einer Datenbankanfrage für eine klinische Datenbank basierend auf den Untersuchungsauftragsdaten, wobei die klinische Datenbank klinische Daten des Patienten umfasst,
- eine Klinische-Daten-Bereitstellungseinheit, ausgebildet zum Bereitstellen der klinischen Daten des Patienten basierend auf der Datenbankanfrage,
- eine Untersuchungsparameter-Ermittlungseinheit, ausgebildet zum Ermitteln des Untersuchungsparameters basierend auf den klinischen Daten.

Eine Ausführungsform der Erfindung sieht ein System vor, ferner aufweisend eine Untersuchungsauftragsdatenbank, welche die Untersuchungsauftragsdaten umfasst, und/oder die klinische Datenbank. Die Untersuchungsauftragsdatenbank kann beispielsweise ein Radiologie-Informationssystem sein und/oder aufweisen.

Eine Ausführungsform der Erfindung sieht ein System vor, ferner aufweisend eine medizinische Bildgebungsvorrichtung, welche zum Ausführen der medizinischen Bildgebungsuntersuchung ausgebildet ist.

Eine Ausführungsform der Erfindung sieht ein System vor, ausgebildet zum Ausführen eines Verfahrens nach einem oder mehreren der in dieser Anmeldung offenbarten Aspekte.

Die Erfindung betrifft ferner ein Computerprogrammprodukt mit einem Computerprogramm, welches direkt in eine Speichereinrichtung eines Computers ladbar ist, mit Programmabschnitten, um alle Schritte eines Verfahrens nach einem oder mehreren der in dieser Anmeldung offenbarten Aspekte auszuführen, wenn das Computerprogramm in dem Computer ausgeführt wird.

Die Erfindung betrifft ferner ein computerlesbares Medium, auf welchem von einem Computer einlesbare und ausführbare Programmabschnitte gespeichert sind, um alle Schritte eines Verfahrens nach einem oder mehreren der in dieser Anmeldung offenbarten Aspekte auszuführen, wenn die Programmabschnitte von dem Computer ausgeführt werden.

Das System zum Ermitteln eines Untersuchungsparameters für eine medizinische Bildgebungsuntersuchung eines Patienten und/oder eine oder mehrere Komponenten des Systems zum Ermitteln eines Untersuchungsparameters für eine medizinische Bildgebungsuntersuchung eines Patienten können von einem Datenverarbeitungssystem gebildet sein. Das Datenverarbeitungssystem kann beispielsweise eine oder mehrere Komponenten in Form von Hardware und/oder eine oder mehrere Komponenten in Form von Software aufweisen. Das Datenverarbeitungssystem kann beispielsweise zumindest teilweise von einem Cloud-Computing-System gebildet sein. Das Datenverarbeitungssystem kann beispielsweise ein Cloud-Computing-System, ein Computernetzwerk, ein Computer, ein Tabletcomputer, ein Smartphone oder ähnliches oder eine Kombination davon sein und/oder aufweisen.

Die Hardware kann beispielsweise mit einer Software zusammenwirken und/oder mittels einer Software konfigurierbar sein. Die Software kann beispielsweise mittels der Hardware ausgeführt werden. Bei der Hardware kann es sich beispielsweise um ein Speichersystem, ein FPGA-System (Field-programmable gate array), ein ASIC-System (Application-specific integrated circuit), ein Mikrocontroller-System, ein Prozessorsystem und Kombinationen davon handeln. Das Prozessorsystem kann beispielsweise einen Mikroprozessor und/oder mehrere zusammenwirkende Mikroprozessoren aufweisen.

Insbesondere kann eine Komponente des Systems nach einem oder mehreren der Aspekte, die in dieser Anmeldung offenbart sind, welche dazu ausgebildet ist, einen gegebenen Schritt eines Verfahrens nach einem oder mehreren der Aspekte, die in dieser Anmeldung offenbart sind, auszuführen, in Form einer Hardware implementiert sein, welche zum Ausführen des gegebenen Schritts konfiguriert ist und/oder welche zum Ausführen einer computerlesbaren Anweisung derart konfiguriert ist, dass die Hardware mittels der computerlesbaren Anweisung zum Ausführen des gegebenen Schritts konfigurierbar ist. Die Schritte des Verfahrens können beispielsweise in einem Prozessor, insbesondere in Form von Berechnungen, ausgeführt werden. Insbesondere kann das System einen Speicherbereich, beispielsweise in Form eines computerlesbaren Mediums, aufweisen, in welchem computerlesbare Anweisungen, beispielsweise in Form eines Computerprogramms, gespeichert sind.

Ein Datentransfer zwischen Komponenten des Datenverarbeitungssystems kann beispielsweise jeweils mittels einer geeigneten Datentransfer-Schnittstelle erfolgen. Die Datentransfer-Schnittstelle zum Datentransfer an und/oder von einer Komponente des Datenverarbeitungssystems kann zumindest teilweise in Form von Software und/oder zumindest teilweise in Form von Hardware realisiert sein. Die Datentransfer-Schnittstelle kann beispielsweise zum Abspeichern von Daten in und/oder zum Laden von Daten aus einem Bereich des Speichersystems ausgebildet sein, wobei auf diesen Bereich des Speichersystems eine oder mehrere Komponenten des Datenverarbeitungssystems zugreifen können.

Insbesondere können Daten, beispielsweise die Untersuchungsauftragsdaten, die Datenbankanfrage, die Sensordaten, die klinischen Daten und/oder der Untersuchungsparameter bereitgestellt werden, indem die Daten geladen, beispielsweise aus einem Bereich eines Speichersystems geladen, und/oder erzeugt, beispielsweise mittels einer medizinischen Bildgebungsvorrichtung erzeugt, werden. Das Computerprogramm ist in das Speichersystem des Datenverarbeitungssystems ladbar und von dem Prozessorsystem des Datenverarbeitungssystems ausführbar. Das Datenverarbeitungssystem kann beispielsweise mittels des Computerprogramms derart ausgebildet sein, dass das Datenverarbeitungssystem die Schritte eines Verfahrens nach einer der Ausführungsformen, die in dieser Anmeldung offenbart sind, ausführen kann, wenn das Computerprogramm von dem Datenverarbeitungssystem ausgeführt wird. Die Schritte des Verfahrens können insbesondere automatisch, beispielsweise ohne Benutzer-Interaktion oder ohne wesentliche Benutzer-Interaktion, ausgeführt werden. Das Verfahren kann ferner Schritte umfassen, bei denen eine Benutzer-Interaktion ausgeführt wird, beispielsweise ein automatisches Ausführen eines Verfahrensschritts manuell gestartet wird und/oder ein automatisch ermitteltes Ergebnis für die weitere Verwendung manuell angepasst wird.

Das Computerprogrammprodukt kann beispielsweise das Computerprogramm sein oder neben dem Computerprogramm mindestens einen zusätzlichen Bestandteil umfassen. Der mindestens eine zusätzliche Bestandteil des Computerprogrammprodukts kann als Hardware und/oder als Software ausgebildet sein. Das Computerprogrammprodukt kann beispielsweise ein Speichermedium, auf dem zumindest ein Teil des Computerprogrammprodukts gespeichert ist, und/oder ein Schlüssel zur Authentifizierung eines Benutzers des Computerprogrammprodukts, insbesondere in Form eines Dongles, aufweisen. Das Computerprogrammprodukt und/oder das Computerprogrammkann beispielsweise ein Cloud-Anwendungs-Programm aufweisen, welches zum Verteilen von Programmabschnitten des Computerprogramms auf verschiedene Verarbeitungseinheiten, insbesondere verschiedene Computer, eines Cloud-Computing-Systems ausgebildet ist, wobei jede der Verarbeitungseinheiten zum Ausführen eines oder mehrerer Programmabschnitte des Computerprogramms ausgebildet ist.

Auf dem computerlesbaren Medium kann beispielsweise das Computerprogrammprodukt nach einer der Ausführungsformen, die in dieser Anmeldung offenbart sind, und/oder das Computerprogramm nach einer der Ausführungsformen, die in dieser Anmeldung offenbart sind, gespeichert sein. Das computerlesbare Medium kann beispielsweise ein Memorystick, eine Festplatte oder ein sonstiger Datenträger sein, der insbesondere lösbar mit dem Datenverarbeitungssystem verbunden oder fest in das Datenverarbeitungssystem integriert sein kann. Das computerlesbare Medium kann beispielsweise einen Bereich des Speichersystems des Datenverarbeitungssystems bilden.

Unter einem Maschinenlernalgorithmus und unter einem trainierten Algorithmus wird im Kontext dieser Anmeldung insbesondere jeweils ein Algorithmus, der zum Maschinellen Lernen ausgebildet ist, verstanden. Ein Maschinenlernalgorithmus kann beispielsweise mit Hilfe von Entscheidungsbäumen, mathematischen Funktionen und/oder allgemeinen Programmiersprachen realisiert werden. Der Maschinenlernalgorithmus kann beispielsweise zum überwachten Lernen und/oder zum unüberwachten Lernen ausgebildet sein. Der Maschinenlernalgorithmus kann beispielsweise zum tiefen Lernen ("deep learning") und/oder zum bestärkendem Lernen ("reinforcement learning") und/oder zum Marginal Space Learning ausgebildet sein. Insbesondere beim überwachten Lernen kann eine Funktionenklasse verwendet werden, welche beispielsweise auf Entscheidungsbäumen ("decision trees"), einem Random Forest, einer logistischen Regression, einer Support Vector Machine, einem künstlichen neuronalen Netz, einer Kernel-Methode, Bayes-Klassifikatoren oder ähnlichem oder Kombinationen davon basiert. Mögliche Implementierungen des Maschinenlernalgorithmus können beispielsweise künstliche Intelligenz verwenden. Berechnungen, insbesondere beim Trainieren eines Maschinenlernalgorithmus, können beispielsweise mittels eines Prozessorsystems ausgeführt werden. Das Prozessorsystem kann beispielsweise einen oder mehrere Prozessoren, insbesondere Grafikprozessoren, aufweisen.

Die Untersuchungsauftragsdaten können insbesondere eine Information in Bezug auf eine medizinische Bildgebungsvorrichtung, mittels welcher die medizinische Bildgebungsuntersuchung des Patienten gemäß den Untersuchungsauftragsdaten durchzuführen ist, umfassen. Die medizinische Bildgebungsuntersuchung kann insbesondere mittels einer medizinischen Bildgebungsvorrichtung, beispielsweise basierend auf dem Untersuchungsparameter, durchgeführt werden. Mittels der medizinischen Bildgebungsvorrichtung kann insbesondere ein Untersuchungsergebnis der medizinischen Bildgebungsuntersuchung des Patienten erzeugt werden.

Die medizinische Bildgebungsvorrichtung kann beispielsweise aus der Bildgebungsmodalitäten-Gruppe gewählt sein, welche aus einem Röntgengerät, einem C-Bogen-Röntgengerät, einem Computertomographiegerät (CT-Gerät), einem Molekularbildgebungsgerät (MI-Gerät), einem Einzelphotonen-Emissions-Computertomographiegerät (SPECT-Gerät), einem Positronen-Emissions-Tomographiegerät (PET-Gerät), einem Magnetresonanztomographiegerät (MR-Gerät) und Kombinationen daraus, insbesondere einem PET-CT-Gerät und einem PET-MR-Gerät, besteht. Die medizinische Bildgebungsvorrichtung kann ferner eine Kombination einer Bildgebungsmodalität, die beispielsweise aus der Bildgebungsmodalitäten-Gruppe gewählt ist, und einer Bestrahlungsmodalität aufweisen. Dabei kann die Bestrahlungsmodalität beispielsweise eine Bestrahlungseinheit zur therapeutischen Bestrahlung aufweisen. Die medizinische Bildgebungsvorrichtung kann insbesondere zumindest ein Zubehörgerät, beispielsweise eine Kontrastmittelinjektionsvorrichtung, und/oder zumindest eine Sensoreinheit zum Erfassen von Sensordaten des Patienten, beispielsweise ein Elektrokardiogramm-Gerät und/oder eine Kamera, insbesondere eine 3D-Kamera, aufweisen.

Ohne Einschränkung des allgemeinen Erfindungsgedankens wird bei einigen der Ausführungsformen ein Computertomographiegerät beispielhaft für eine medizinische Bildgebungsvorrichtung genannt.

Gemäß einer Ausführungsform der Erfindung weist die medizinische Bildgebungsvorrichtung eine Akquisitionseinheit, welche zur Akquisition der Akquisitionsdaten ausgebildet ist, auf. Insbesondere kann die Akquisitionseinheit eine Strahlungsquelle und einen Strahlungsdetektor aufweisen.

Eine Ausführungsform der Erfindung sieht vor, dass die Strahlungsquelle zur Emission und/oder zur Anregung einer Strahlung, insbesondere einer elektromagnetischen Strahlung, ausgebildet ist und/oder dass der Strahlungsdetektor zur Detektion der Strahlung, insbesondere der elektromagnetischen Strahlung, ausgebildet ist. Die Strahlung kann beispielsweise von der Strahlungsquelle zu einem abzubildenden Bereich gelangen und/oder nach einer Wechselwirkung mit dem abzubildenden Bereich zu dem Strahlungsdetektor gelangen. Bei der Wechselwirkung mit dem abzubildenden Bereich wird die Strahlung modifiziert und damit zum Träger von Informationen, die den abzubildenden Bereich betreffen. Bei der Wechselwirkung der Strahlung mit dem Detektor werden diese Informationen in Form von Akquisitionsdaten erfasst.

Insbesondere bei einem Computertomographiegerät und bei einem C-Bogen-Röntgengerät können die Akquisitionsdaten Projektionsdaten, die Akquisitionseinheit eine Projektionsdaten-Akquisitionseinheit, die Strahlungsquelle eine Röntgenquelle, der Strahlungsdetektor ein Röntgendetektor sein. Der Röntgendetektor kann insbesondere ein quantenzählender und/oder energieauflösender Röntgendetektor sein.

Insbesondere bei einem Magnetresonanztomographiegerät können die Akquisitionsdaten ein Magnetresonanzdatensatz, die Akquisitionseinheit eine Magnetresonanzdaten-Akquisitionseinheit, die Strahlungsquelle eine erste Hochfrequenzantenneneinheit, der Strahlungsdetektor die erste Hochfrequenzantenneneinheit und/oder eine zweite Hochfrequenzantenneneinheit sein.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der eine Vorrichtung betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Neben den in dieser Anmeldung ausdrücklich beschriebenen Ausführungsformen der Erfindung sind vielfältige weitere Ausführungsformen der Erfindung denkbar, zu denen der Fachmann gelangen kann, ohne den Bereich der Erfindung zu verlassen, soweit er durch die Ansprüche vorgegeben ist.

Die Verwendung der unbestimmten Artikel "ein" bzw. "eine" schließt nicht aus, dass das betroffene Merkmal auch mehrfach vorhanden sein kann. Die Verwendung des Ausdrucks "aufweisen" schließt nicht aus, dass die mittels des Ausdrucks "aufweisen" verknüpften Begriffe identisch sein können. Beispielsweise weist die medizinische Bildgebungsvorrichtung die medizinische Bildgebungsvorrichtung auf. Die Verwendung des Ausdrucks "Einheit" schließt nicht aus, dass der Gegenstand, auf den sich der Ausdruck "Einheit" bezieht, mehrere Komponenten aufweisen kann, die räumlich voneinander separiert sind.

Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.

Es zeigen:
Fig. 1 ein Ablaufdiagramm eines Verfahrens zum Ermitteln eines Untersuchungsparameters für eine medizinische Bildgebungsuntersuchung,
Fig. 2 ein System zum Ermitteln eines Untersuchungsparameters für eine medizinische Bildgebungsuntersuchung,
Fig. 3 ein System zum Ermitteln eines Untersuchungsparameters für eine medizinische Bildgebungsuntersuchung, und
Fig. 4 eine medizinische Bildgebungsvorrichtung.

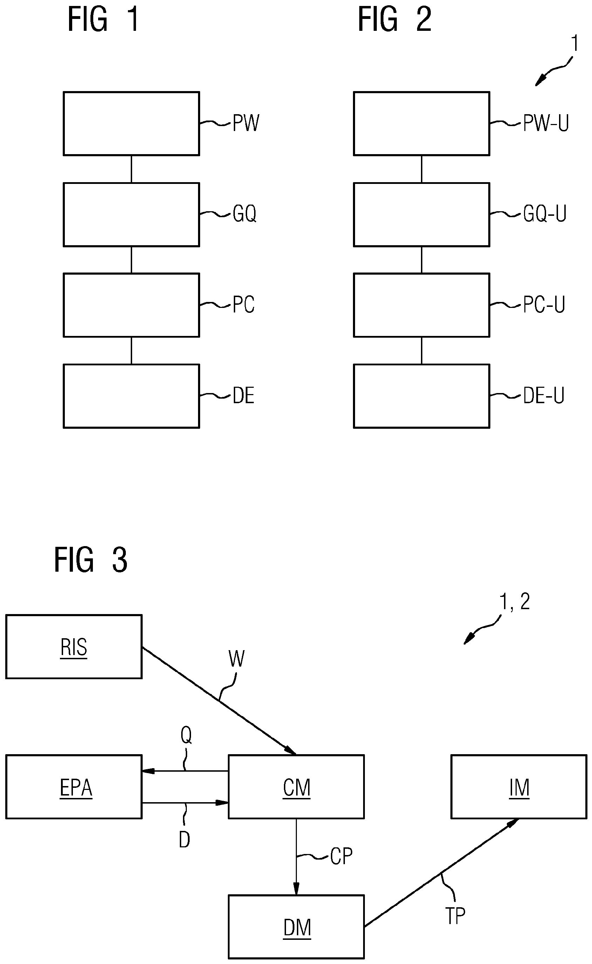

Die Fig. 1 zeigt ein Ablaufdiagramm eines Verfahrens zum Ermitteln eines Untersuchungsparameters TP für eine medizinische Bildgebungsuntersuchung eines Patienten 13, wobei das Verfahren die folgenden Schritte umfasst:
- Bereitstellen PW von Untersuchungsauftragsdaten W, welche die medizinische Bildgebungsuntersuchung des Patienten 13 betreffen,
- Generieren GQ einer Datenbankanfrage Q für eine klinische Datenbank EPA basierend auf den Untersuchungsauftragsdaten W, wobei die klinische Datenbank EPA klinische Daten D des Patienten 13 umfasst,
- Bereitstellen PC der klinischen Daten D des Patienten 13 basierend auf der Datenbankanfrage Q,
- Ermitteln DE des Untersuchungsparameters TP basierend auf den klinischen Daten D.

Die Fig. 2 zeigt ein System 1 zum Ermitteln eines Untersuchungsparameters TP für eine medizinische Bildgebungsuntersuchung eines Patienten 13, aufweisend:
- eine Untersuchungsauftrag-Bereitstellungseinheit PW-U, ausgebildet zum Bereitstellen PW von Untersuchungsauftragsdaten W, welche die medizinische Bildgebungsuntersuchung des Patienten 13 betreffen,
- eine Generierungseinheit GQ-U, ausgebildet zum Generieren GQ einer Datenbankanfrage Q für eine klinische Datenbank EPA basierend auf den Untersuchungsauftragsdaten W, wobei die klinische Datenbank EPA klinische Daten D des Patienten 13 umfasst,
- eine Klinische-Daten-Bereitstellungseinheit PC-U, ausgebildet zum Bereitstellen PC der klinischen Daten D des Patienten 13 basierend auf der Datenbankanfrage Q,
- eine Untersuchungsparameter-Ermittlungseinheit DE-U, ausgebildet zum Ermitteln DE des Untersuchungsparameters TP basierend auf den klinischen Daten D.

Die Fig. 3 zeigt ein System 1 zum Ermitteln eines Untersuchungsparameters für eine medizinische Bildgebungsuntersuchung eines Patienten 13, aufweisend ein Radiologie-Informationssystem RIS als Untersuchungsauftragsdatenbank, eine klinische Datenbank EPA, ein Verbindungsmodul CM, ein Entscheidungsmodul DM und eine medizinische Bildgebungsvorrichtung 2. Beispielsweise kann das Verbindungsmodul CM und/oder das Entscheidungsmodul DM als Teil der medizinischen Bildgebungsvorrichtung 2, insbesondere als ein Teil der Steuerungsvorrichtung 30 ausgebildet sein. Alternativ kann das Verbindungsmodul CM und/oder das Entscheidungsmodul beispielsweise von einer Datenverarbeitungseinheit, die nicht ein Teil der medizinischen Bildgebungsvorrichtung 2 ist, gebildet sein. Gemäß einer weiteren Ausführungsform bilden die Steuerungsvorrichtung 30 zusammen mit einer Datenverarbeitungseinheit, die nicht ein Teil der medizinischen Bildgebungsvorrichtung 2 ist, zusammen das Verbindungsmodul CM und/oder das Entscheidungsmodul DM. Insbesondere kann das Verbindungsmodul CM und/oder das Entscheidungsmodul DM von einem Datenverarbeitungssystem nach einem der in dieser Anmeldung beschriebenen Aspekte gebildet sein. Insbesondere können das Verbindungsmodul CM und/oder das Entscheidungsmodul DM in Form von Software und/oder Hardware realisiert sein.

In dem Radiologie-Informationssystem RIS sind Untersuchungsauftragsdaten W, beispielsweise in Form eines Eintrages in einer radiologischen Worklist, hinterlegt. Die Untersuchungsauftragsdaten W werden von dem Radiologie-Informationssystem RIS an das Verbindungsmodul CM übertragen. Beispielsweise kann das Radiologie-Informationssystem RIS die Untersuchungsauftrag-Bereitstellungseinheit PW-U aufweisen, wobei die Untersuchungsauftragsdaten W bereitgestellt werden, indem die Untersuchungsauftragsdaten W mittels der Untersuchungsauftrag-Bereitstellungseinheit PW-U geladen und/oder gesendet werden. Alternativ kann beispielsweise das Verbindungsmodul CM die Untersuchungsauftrag-Bereitstellungseinheit PW-U aufweisen, wobei die Untersuchungsauftragsdaten W bereitgestellt werden, indem die Untersuchungsauftragsdaten W mittels der Untersuchungsauftrag-Bereitstellungseinheit PW-U geladen und/oder empfangen werden.

Die klinische Datenbank EPA umfasst klinische Daten D des Patienten 13. Die klinischen Daten D des Patienten 13 liegen zumindest teilweise in Form von Einträgen in einer elektronischen Patientenakte des Patienten 13 vor. Basierend auf den Untersuchungsauftragsdaten W kann das Verbindungsmodul CM auf die klinische Datenbank EPA, insbesondere auf die elektronische Patientenakte des Patienten 13, zugreifen und/oder aus der klinischen Datenbank EPA, insbesondere aus den klinischen Daten D, Informationen extrahieren, welche für die medizinische Bildgebungsuntersuchung relevant sind. Insbesondere kann die Datenbankanfrage Q von dem Verbindungsmodul CM and die klinische Datenbank EPA übertragen werden. Insbesondere können die klinische Daten D von der klinischen Datenbank EPA an das Verbindungsmodul CM übertragen werden.

Die Informationen, welche für die medizinische Bildgebungsuntersuchung relevant sind, können dabei in Abhängigkeit von den Untersuchungsauftragsdaten W ermittelt werden. Insbesondere kann das Verbindungsmodul CM basierend auf den Untersuchungsauftragsdaten W eine Annahme über ein erwartetes Ergebnis der medizinischen Bildgebungsuntersuchung des Patienten 13 generieren und/oder insbesondere basierend auf der Annahme klinische Daten D aus der klinischen Datenbank EPA automatisch abfragen. Insbesondere kann das Verbindungsmodul CM die Generierungseinheit GQ-U aufweisen.

Basierend auf den klinischen Daten D kann der Untersuchungsparameter TP ermittelt werden. Der Untersuchungsparameter TP kann beispielsweise ein technischer Parameter sein, der eine Bilddatenakquisitionseinheit IM, zum Beispiel mit einer Strahlungsquelle 26 und/oder mit einem Strahlungsdetektor 28, der medizinischen Bildgebungsvorrichtung 2 betrifft. Die Klinische-Daten-Bereitstellungseinheit PC-U kann beispielsweise als Teil der klinischen Datenbank EPA und/oder als Teil des Verbindungsmoduls CM ausgebildet sein.

Das Verbindungsmodul CM und/oder das Entscheidungsmodul DM können jeweils Methoden der künstlichen Intelligenz verwenden. Das System 1 kann optional eine Rückkopplung (Feedback Loop) umfassen, wobei ein Untersuchungsergebnis der medizinischen Bildgebungsuntersuchung des Patienten 13 verwendet wird, um die künstliche Intelligenz des Verbindungsmoduls CM und/oder die künstliche Intelligenz des Entscheidungsmoduls DM zu optimieren. Dabei kann das Untersuchungsergebnis automatisch von einer Software oder von einem Benutzer, insbesondere von einem Befunder, bewertet werden. Das System 1 kann somit als ein kontinuierliches selbstlernendes System, insbesondere basierend auf verstärkendem Lernen, ausgebildet sein und/oder zur kontinuierlichen Anpassung an die Bedürfnisse des Benutzers ausgebildet sein. Dabei kann der Benutzer beispielsweise bestimmte Strategien für das Lernen vorgeben, zum Beispiel indem eine niedrige Strahlendosis oder eine kurze Dauer der medizinischen Bildgebungsuntersuchung stärker belohnt werden.

Das Verbindungsmodul CM und/oder das Entscheidungsmodul DM ist zum Strukturieren und Klassifizieren der klinischen Daten D ausgebildet. Beispielsweise kann das Verbindungsmodul CM basierend auf den klinischen Daten D strukturierte und/oder klassifizierte klinische Daten CP ermitteln. Das Entscheidungsmodul DM kann basierend auf den strukturierten und/oder klassifizierten klinische Daten CP den Untersuchungsparameter TP ermitteln. Das Verbindungsmodul CM kann beispielsweise zumindest ein Teilmodul aufweisen, welches zum Strukturieren und/oder Klassifizieren der klinischen Daten D basierend auf einem Datentyp der klinischen Daten D ausgebildet ist. Die Untersuchungsparameter-Ermittlungseinheit DE-U kann beispielsweise zumindest einen Teil des Verbindungsmoduls CM und/oder zumindest einen Teil des Entscheidungsmoduls DM aufweisen.

Gemäß einem ersten Ausführungsbeispiel betreffen die Untersuchungsauftragsdaten W eine Computertomographie-Koronarangiographie für den Patienten 13. Die klinischen Daten D weisen die Diagnose "Angina Pectoris" (ICD I20.0) und "ventrikuläre Extrasystolie" (ICD I49.3) auf. Die klinischen Daten D weisen ferner einen Befund zu einer Ultraschalluntersuchung auf, der die Ejektionsfraktion mit 25%-35% angibt. Aufgrund dieser Angaben schlägt das Entscheidungsmodul DM dem Benutzer ein Untersuchungsprotokoll für die medizinische Bildgebungsuntersuchung mit retrospektiven Gating und verlängertem Intervall zwischen Kontrastmittelinjektion und Bilddatenakquisition vor, da der Bolus aufgrund der Herzinsuffizienz verzögert eintreffen wird.

Gemäß einem zweiten Ausführungsbeispiel betreffen die Untersuchungsauftragsdaten W eine Magnetresonanztomographie-Untersuchung der Lunge des Patienten 13. Die klinischen Daten D weisen ein Anamneseprotokoll auf, in dem der Patient 13 angibt, starker Raucher zu sein und wegen Luftnot keine Treppen steigen zu können. Die künstliche Intelligenz im Verbindungsmodul CM ordnet diesem Freitext die Diagnose ICD J44.- (chronisch obstruktive Lungenkrankheit) zu. Das Entscheidungsmodul DM schlägt darauf basierend ein "Free Breathing" Protokoll vor.

Gemäß einem dritten Ausführungsbeispiel betreffen die Untersuchungsauftragsdaten W eine Magnetresonanztomographie-Untersuchung des Thorax des Patienten 13. Die klinischen Daten D weisen Abrechnungsdaten auf, in denen wiederholt die Ziffer 13552 vorkommt, welcher die Kostenposition Kontrolle Herzschrittmacher zugeordnet ist. Das Entscheidungsmodul DM gibt darauf basierend einen entsprechenden Warnhinweis aus, dass der Patient 13 einen Schrittmacher hat und daher möglicherweise nicht für eine MRT-Untersuchung geeignet ist.

Gemäß einem vierten Ausführungsbeispiel betreffen die Untersuchungsauftragsdaten W eine Computertomographie-Untersuchung der Lunge bei Atemnot. Die klinischen Daten D weisen eine Einweisungsdiagnose des Patienten 13 mit der Klassifikation ICD I26.0 (akute Lungenembolie mit Cor Pulmonale) auf. Das Entscheidungsmodul DM stellt basierend darauf fest, dass eine Darstellung der Lungengefäße des Patienten 13 dringend erforderlich ist, ermittelt ein entsprechendes Untersuchungsprotokoll mit Kontrastmittelgabe und weist den Benutzer darauf hin, die medizinische Bildgebungsuntersuchung dringend durchzuführen.

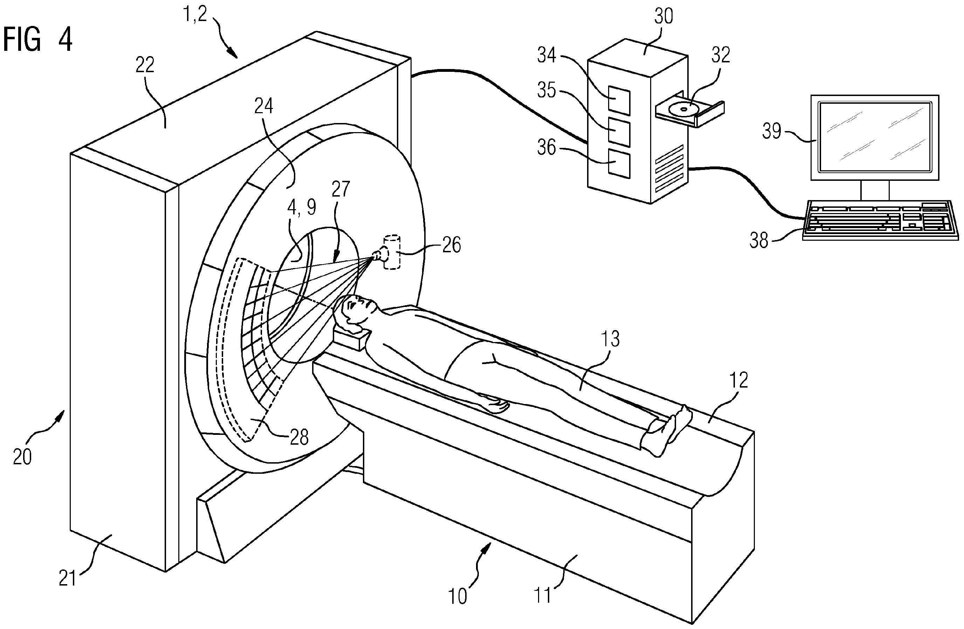

Die Fig. 4 zeigt eine medizinische Bildgebungsvorrichtung 2. Ohne Beschränkung des allgemeinen Erfindungsgedankens ist für die medizinische Bildgebungsvorrichtung 2 beispielhaft ein Computertomographiegerät gezeigt. Die medizinische Bildgebungsvorrichtung 2 weist die Gantry 20, die tunnelförmige Öffnung 9, die Patientenlagerungsvorrichtung 10 und die Steuerungsvorrichtung 30 auf. Die Gantry 20 weist den stationären Tragrahmen 21, den Kipprahmen 22 und den Rotor 24 auf.

Der Kipprahmen 22 ist mittels einer Kipplagerungsvorrichtung an dem stationären Tragrahmen 21 relativ zu dem stationären Tragrahmen 21 kippbar angeordnet. Der Rotor 24 ist mittels einer Drehlagerungsvorrichtung an dem Kipprahmen 22 um eine Rotationsachse relativ zu dem Kipprahmen 22 drehbar angeordnet. In die tunnelförmige Öffnung 9 ist der Patient 13 einführbar. In der tunnelförmigen Öffnung 9 befindet sich der Akquisitionsbereich 4. In dem Akquisitionsbereich 4 ist ein abzubildender Bereich des Patienten 13 derart positionierbar, dass die Strahlung 27 von der Strahlungsquelle 26 zu dem abzubildenden Bereich gelangen kann und nach einer Wechselwirkung mit dem abzubildenden Bereich zu dem Strahlungsdetektor 28 gelangen kann.

Die Patientenlagerungsvorrichtung 10 weist den Lagerungssockel 11 und die Lagerungsplatte 12 zur Lagerung des Patienten 13 auf. Die Lagerungsplatte 12 ist derart relativ zu dem Lagerungssockel 11 bewegbar an dem Lagerungssockel 11 angeordnet, dass die Lagerungsplatte 12 in einer Längsrichtung der Lagerungsplatte 12 in den Akquisitionsbereich 4 einführbar ist. Die medizinische Bildgebungsvorrichtung 2 ist zur Akquisition von Akquisitionsdaten basierend auf einer elektromagnetischen Strahlung 27 ausgebildet. Die medizinische Bildgebungsvorrichtung 2 weist eine Akquisitionseinheit auf. Die Akquisitionseinheit ist eine Projektionsdaten-Akquisitionseinheit mit der Strahlungsquelle 26, z. B. einer Röntgenquelle, und dem Detektor 28, z. B. einem Röntgendetektor, insbesondere einem energieauflösenden Röntgendetektor.

Die Strahlungsquelle 26 ist an dem Rotor 24 angeordnet und zur Emission einer Strahlung 27, z. B. einer Röntgenstrahlung, mit Strahlungsquanten 27 ausgebildet. Der Detektor 28 ist an dem Rotor 24 angeordnet und zur Detektion der Strahlungsquanten 27 ausgebildet. Die Strahlungsquanten 27 können von der Strahlungsquelle 26 zu dem abzubildenden Bereich des Patienten 13 gelangen und nach einer Wechselwirkung mit dem abzubildenden Bereich auf den Detektor 28 auftreffen. Auf diese Weise können mittels der Akquisitionseinheit Akquisitionsdaten des abzubildenden Bereichs in Form von Projektionsdaten erfasst werden.

Die Steuerungsvorrichtung 30 ist zum Empfangen der von der Akquisitionseinheit akquirierten Akquisitionsdaten ausgebildet. Die Steuerungsvorrichtung 30 ist zum Steuern der medizinischen Bildgebungsvorrichtung 2, insbesondere basierend auf dem Untersuchungsparameter TP, ausgebildet. Die Steuerungsvorrichtung 30 weist die Datenverarbeitungseinheit 35, das computerlesbare Medium 32 und das Prozessorsystem 36 auf. Die Steuerungsvorrichtung 30, insbesondere die Datenverarbeitungseinheit 35, wird von einem Datenverarbeitungssystem, welches einen Computer aufweist, gebildet. Die Datenverarbeitungseinheit 35 kann beispielsweise die Komponenten PW-U, GQ-U, PC-U und DE-U des Systems 1 und/oder das Verbindungsmodul CM und/oder das Entscheidungsmodul DM aufweisen.

Die Steuerungsvorrichtung 30 weist die Bildrekonstruktionseinrichtung 34 auf. Mittels der Bildrekonstruktionseinrichtung 34 kann basierend auf den Akquisitionsdaten ein medizinischer Bilddatensatz rekonstruiert werden. Die medizinische Bildgebungsvorrichtung 2 weist eine Eingabevorrichtung 38 und eine Ausgabevorrichtung 39 auf, welche jeweils mit der Steuerungsvorrichtung 30 verbunden sind. Die Eingabevorrichtung 38 ist zum Eingeben von Steuerungs-Informationen, z. B. Bildrekonstruktionsparametern, Untersuchungsparametern oder ähnliches, ausgebildet. Die Ausgabevorrichtung 39 ist insbesondere zum Ausgeben von Steuerungs-Informationen, Bildern und/oder akustischen Signalen ausgebildet.

Die Erfindung ermöglicht damit eine verbesserte Ermittlung von Untersuchungsparametern für eine medizinische Bildgebungsuntersuchung. Insbesondere können auf diese Weise auch mit weniger qualifiziertem Personal optimale klinische Ergebnisse erzeugt werden. Insbesondere ist es nicht erforderlich, dass die klinischen Daten durch den Benutzer im Wesentlichen manuell ausgewählt und/oder zur weiteren automatischen Verarbeitung eingegeben werden. Dadurch können sowohl der Zeitaufwand als auch die Fehleranfälligkeit beim Ermitteln des Untersuchungsparameters verringert werden.

## Patentansprüche

1. Verfahren zum Ermitteln eines Untersuchungsparameters (TP) für eine medizinische Bildgebungsuntersuchung eines Patienten (13), wobei das Verfahren die folgenden Schritte umfasst:
- Bereitstellen (PW) von Untersuchungsauftragsdaten (W), welche die medizinische Bildgebungsuntersuchung des Patienten (13) betreffen,
- Generieren (GQ) einer Datenbankanfrage (Q) für eine klinische Datenbank (EPA) basierend auf den Untersuchungsauftragsdaten (W), wobei die klinische Datenbank (EPA) klinische Daten (D) des Patienten (13) umfasst,
- Bereitstellen (PC) der klinischen Daten (D) des Patienten (13) basierend auf der Datenbankanfrage (Q),
- Ermitteln (DE) des Untersuchungsparameters (TP) basierend auf den klinischen Daten (D).

2. Verfahren nach Anspruch 1, wobei der Untersuchungsparameter (TP) aus der Gruppe gewählt ist, welche aus einem Akquisitionsparameter, einem Rekonstruktionsparameter, einem Bildverarbeitungsparameter, einem Kontrastmittelparameter und Kombinationen davon besteht.

3. Verfahren nach einem der Ansprüche 1 bis 2, wobei die Datenbankanfrage (Q) für eine unscharfe Suche in der klinischen Datenbank (EPA) geeignet ist.

4. Verfahren nach einem der Ansprüche 1 bis 3, wobei die Datenbankanfrage (Q) basierend auf künstlicher Intelligenz generiert wird.

5. Verfahren nach einem der Ansprüche 1 bis 4, wobei die klinischen Daten (D) basierend auf künstlicher Intelligenz strukturiert und/oder klassifiziert werden.

6. Verfahren nach einem der Ansprüche 1 bis 5, wobei der Untersuchungsparameter (TP) basierend auf den klinischen Daten (D) mittels eines trainierten Untersuchungsparameter-Ermittlungs-Algorithmus, welcher auf maschinellem Lernen basiert, ermittelt wird.

7. Verfahren nach einem der Ansprüche 1 bis 6,
- wobei die medizinische Bildgebungsuntersuchung des Patienten (13) basierend auf dem Untersuchungsparameter (TP) durchgeführt wird,
- wobei ein Untersuchungsergebnis der medizinischen Bildgebungsuntersuchung des Patienten (13) erzeugt wird,
- wobei ein Trainingsdatensatz mit einem Trainingsinput und einem Trainingsoutput zum Trainieren eines Maschinenlernalgorithmus erzeugt wird,
- wobei der Trainingsinput die klinischen Daten (D) und/oder die strukturierten und/oder klassifizierten klinischen Daten (D) aufweist,
- wobei der Trainingsoutput das Untersuchungsergebnis aufweist.

8. Verfahren nach Anspruch 7,
- wobei basierend auf dem Trainingsdatensatz ein Maschinenlernalgorithmus trainiert wird, welcher zum Generieren der Datenbankanfrage (Q) für die klinische Datenbank (EPA) basierend auf den Untersuchungsauftragsdaten (W) und/oder zum Strukturieren und/oder Klassifizieren der klinischen Daten (D) und/oder zum Ermitteln des Untersuchungsparameters (TP) basierend auf den klinischen Daten (D) ausgebildet ist.

9. Verfahren nach einem der Ansprüche 1 bis 8,
- wobei zumindest ein Abrechenbarkeitskriterium bereitgestellt wird,
- wobei basierend auf den Untersuchungsauftragsdaten (W) und/oder basierend auf den klinischen Daten (D) und/oder basierend auf den strukturierten und/oder klassifizierten klinischen Daten (D) und/oder basierend auf dem Untersuchungsparameter (TP) eine Überprüfung der medizinischen Bildgebungsuntersuchung in Bezug auf das zumindest eine Abrechenbarkeitskriterium durchgeführt wird.

10. System (1) zum Ermitteln eines Untersuchungsparameters (TP) für eine medizinische Bildgebungsuntersuchung eines Patienten (13), aufweisend:
- eine Untersuchungsauftrag-Bereitstellungseinheit, ausgebildet zum Bereitstellen (PW) von Untersuchungsauftragsdaten (W), welche die medizinische Bildgebungsuntersuchung des Patienten (13) betreffen,
- eine Generierungseinheit (GQ-U), ausgebildet zum Generieren einer Datenbankanfrage (Q) für eine klinische Datenbank (EPA) basierend auf den Untersuchungsauftragsdaten (W), wobei die klinische Datenbank (EPA) klinische Daten (D) des Patienten (13) umfasst,
- eine Klinische-Daten-Bereitstellungseinheit (PC-U), ausgebildet zum Bereitstellen (PC) der klinischen Daten (D) des Patienten (13) basierend auf der Datenbankanfrage (Q),
- eine Untersuchungsparameter-Ermittlungseinheit (DE-U), ausgebildet zum Ermitteln (DE) des Untersuchungsparameters (TP) basierend auf den klinischen Daten (D).

11. System (1) nach Anspruch 10, ferner aufweisend eine Untersuchungsauftragsdatenbank (RIS), welche die Untersuchungsauftragsdaten (W) umfasst, und/oder die klinische Datenbank (EPA) .

12. System (1) nach einem der Ansprüche 10 bis 11, ferner aufweisend eine medizinische Bildgebungsvorrichtung (2), welche zum Ausführen der medizinischen Bildgebungsuntersuchung ausgebildet ist.

13. System (1) nach einem der Ansprüche 10 bis 12, ausgebildet zum Ausführen eines Verfahrens nach einem der Ansprüche 1 bis 9.

14. Computerprogrammprodukt mit einem Computerprogramm, welches direkt in eine Speichereinrichtung eines Computers (30) ladbar ist, mit Programmabschnitten, um alle Schritte eines Verfahrens nach einem der Ansprüche 1 bis 9 auszuführen, wenn das Computerprogramm in dem Computer (30) ausgeführt wird.

15. Computerlesbares Medium (32), auf welchem von einem Computer (30) einlesbare und ausführbare Programmabschnitte gespeichert sind, um alle Schritte eines Verfahrens nach einem der Ansprüche 1 bis 9 auszuführen, wenn die Programmabschnitte von dem Computer (30) ausgeführt werden.
